# EUROPEAN PATENT SPECIFICATION

(11) **EP 2 031 501 B1**
(45) Date of publication and mention of the grant of the patent: **05.08.2015**
(21) Application number: 08011144.6
(22) Date of filing: 19.06.2008
(51) Int. Cl.: C12M 3/00

(54) **Apparatus for the culture and growth of cells to a three dimensional tissue**
Vorrichtung zur Kultur und Wachstum von Zellen auf einem dreidimensionalen Gewebe
Appareil pour la culture et la croissance de cellules pour un tissu tridimensionnel

(30) Priority: 27.08.2007 US 895645
(43) Date of publication of application: 04.03.2009
(73) Proprietor: Biomimetics Technologies Inc., Toronto, ON M6S 2X4 (CA)
(72) Inventor: Gille, Christoph, 16540 Hohen Neuendorf (DE); Holm, Chris, 42105 Wuppertal (DE); Israelowitz, Meir, Toronto Ontario M4K 1J9 (CA); Rizvi, Seyed W. H., Toronto Ontario M6S 2X4 (CA); von Schroeder, Herbert P., Toronto Ontario M6S 2X4 (CA)
(74) Representative: Jabbusch, Matthias

(56) References cited:
- WO-A-2005/056747
- WO-A-2006/088029
- WO-A-2006/122088

## Description

The invention relates to an apparatus and a method for the culture and growth of cells to a three dimensional tissue.

### Background of the Invention

Known in the art is the bioreactor of the document WO 2005/ 056747. This is an apparatus for the culture and growth of cells to a three dimensional tissue, consisting of a laminar flow reactor, in which the growing of the cells takes place on a grid in the midth of a housing, a pump, a storage tank for a nutrient fluid and pipes connecting these parts to create a circulation of the nutrient fluid through these parts. For the growth of a cell mass it is very important to hold the flow in a laminar flowing. Because it is not possible to see from the outside if there is a laminar or a turbulent flow around the cell mass it is necessay to work under the possible maximum of the laminar flow. Under this condition the growth takes much time. For medical purposes it is mostly necessary to produce the growing cell mass in a required short time.

### Object of the invention

is to build an apparatus for the culture and growth of cells in a homogenous thickness over a wide plain (surface) to a three dimensional tissue to be used e.g. in hospitals

### Description of the Invention

The invention consist in a method for the growth of cells to a three dimensional tissue, in which biomaterial is taken on a grid , the grid is put in the midth of a laminar flow of a nutrient and the grid is brought in the midth of a circle of bypasses. For the growing of the cells it is important, that the flow through the mass of growing cells is steady and laminar. The bypasses are closed or opened to controll the flow to have a permanent laminar flow through the grid with its cells at the highest level without turbulences.

The apparatus for the culture and growth of cells to a three dimensional tissue of the invention consists of a laminar flow reactor, in which the growing of the cells takes place, a pump, a storage tank for a nutrient fluid and pipes connecting these parts to create a permanent circulation of the nutrient fluid through these parts. In this apparatus is the grid in the grid carrying chamber surrounded by a plurality of bypasses. With these bypasses it is possible to get a constant laminar flow in the whole area of the grid and it is further on possible to reach the maximum of the laminar flow without turbulences in this flow.

This is to obtain in an apparatus with a reactor whoose housing consists of three parts, two shells each with a hole used for an entrance or an outlet of the nutrient fluid, and between theese shells a chamber carrying a grid to support the biomaterial surrounded by a plurality of bypasses.

For this purpose it is important in this apparatus that the plurality of bypasses are controlled by a handle or a motor to close the bypasses partly or completly to steer the run through of the nutrient fluid through the cellmass.

For this purpose it is also advantageous to have a grid in the form of a circle surounded by the channels of the bypasses.

In this case a slidable ring with the same scale of canals of the by-passes can be installed over the bypasses to cover and close the bypasses partly or completly.

In this apparatus the bypasses can have the form of an iris-diaphragm and an irisclamp to control the flow through theese bypasses.

The shells of the reactor have each a flange to fasten one shell to the other shell and include between them the grid or the grid carring chamber.

The shells include a big volume of fluid to create an equal flow of the fluid through the chamber between the two shells.

In the housing of the reactor of this Installation one or more windows can be installed in the walls of the reactor showing the kind of flow and the growing of the cells.

Another design of this apparatus has a grid carring a frame on the surface on the grid surrounding the center part of the grid for the growing of the cells und a another part surroundig the center part of the grid acting as a bypass.

The apparatus can be equipped with a device for a simple take out the grid with the grown cell mass and to bring in a new grid for the growing cell mass located between the upper and the under shell consisting of a holder to carry a slidable carrier of the grid for growing the cell masses and the surrounding bypasses and consisting of an devive for moving the upper and the under shell away from the holder (to open the apparatus for taking out the grid with the grown cell mass) and to press the to shells against the holder (to close the apparatus for its working phase).

The carrier has on its upper side and on its under side sealings surrounding the grid and the bypasses to seal the carrier with the shells of the apparatus. The upper shell is slidable against the under shell in a direction of the flow of the nutrient fluid for a small distance to give the possibility to take out the carrier and to fill in a new carrier. The magnitude of the diameter of the canals is controlled by the motor for adjusting. It can have hydraulic means which press the shells and the sealings together in the growing phase of the cell mass to tighten the apparatus and which lift the upper shell from the under shell at the end of the growing process to give a possibility to take out the carrier out of the inner room between the two shells (it is the reactor room for the growing process) in an easy way.

### Descrption of the Drawings

- Fig.1: shows a block diagramm of the installation for the culture and growth, of cells to a three dimensional tissue.
- Fig 2: shows a crosssection through the laminar flow reactor with a grid for growing cells surounded by bypasses.
- Fig.3: shows a view of the iris valve of a laminar flow reactor.
- Fig.4: shows a grid with a growing area in the midth and a surrounding bypass area.
- Fig.5: shows a device for take out and bring in the grid carrier.
- Fig.6: shows the grid carrier of Fig.5.
- Fig.7: a shelter over the grid with six holes to observe the growth

### Detailed Description of the drawings

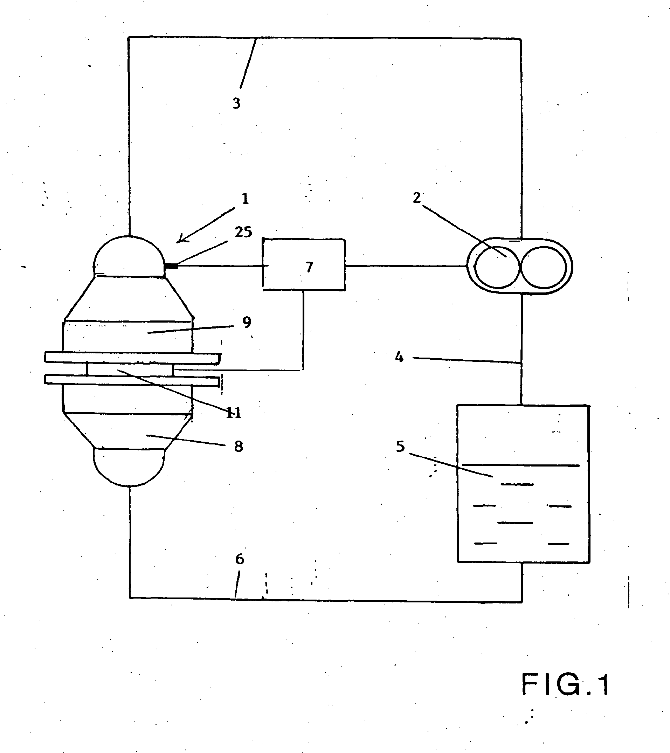

In the block diagramm of Fig. 1 the laminar flow reactor 1 is connected with pump 2 by the pipe 3, pipe 4 connects pump 3 with the storage tank 5 for the nutrient fluid and this tank is connected with the reactor 1 by pipe 6. The nutrient fluid flows in a circle from the tank 5 to the reactor1, from the reactor 1 to the pump 2 and from the pump 2 bach to the tank 5.

A control box 7 controlls the velocity of the fluid in the pipes and holds the laminar flow in the reactor.

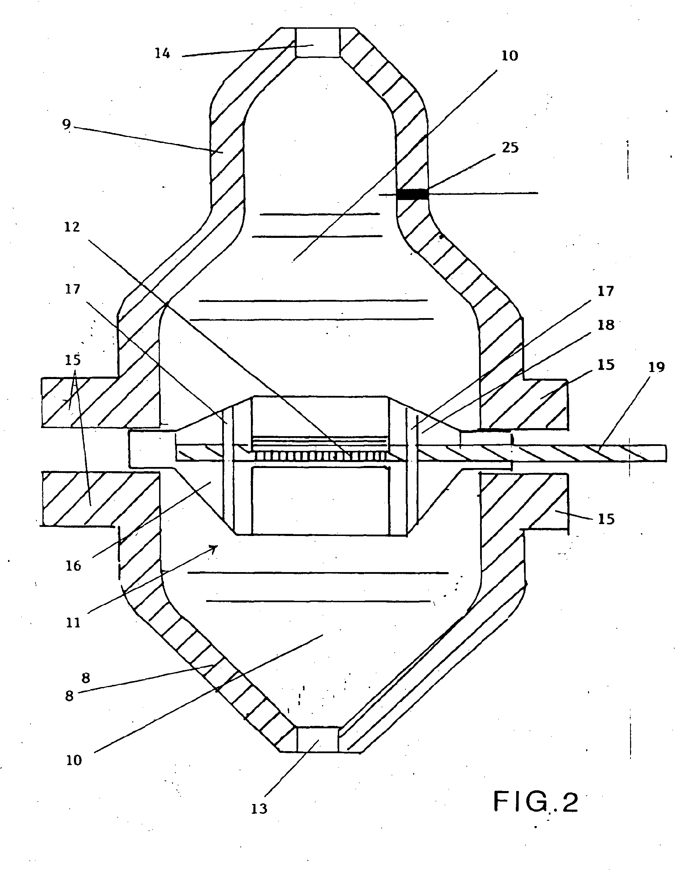

In Fig.2 is shown a crosssection of the reactor. Two shells 8,9 with a big interior 10 include and carry a chamber 11 with the grid 12 carrying the biomaterial. Each shell 8,9 has on its end a hole 13,14 connected with the pipes 3 and 4. In the frame 15 of the chamber 11 are in a circle many canals 17 used as bypasses. Theese are interrupted by a ring 18 with the same canals 17. By rotating the ring 18 with the handle 19 the bypasses are covered and closed partly or completly or are opend again.

In this way can be obtained a continous laminar flow of the nutrient fluid through the reactor: The big inner of the shells 8,9 causes a very slow flow in the shells 8,9, the bypasses 17 surroundig the grid 12 avoid turbulences of the fluid passing the grid 12.

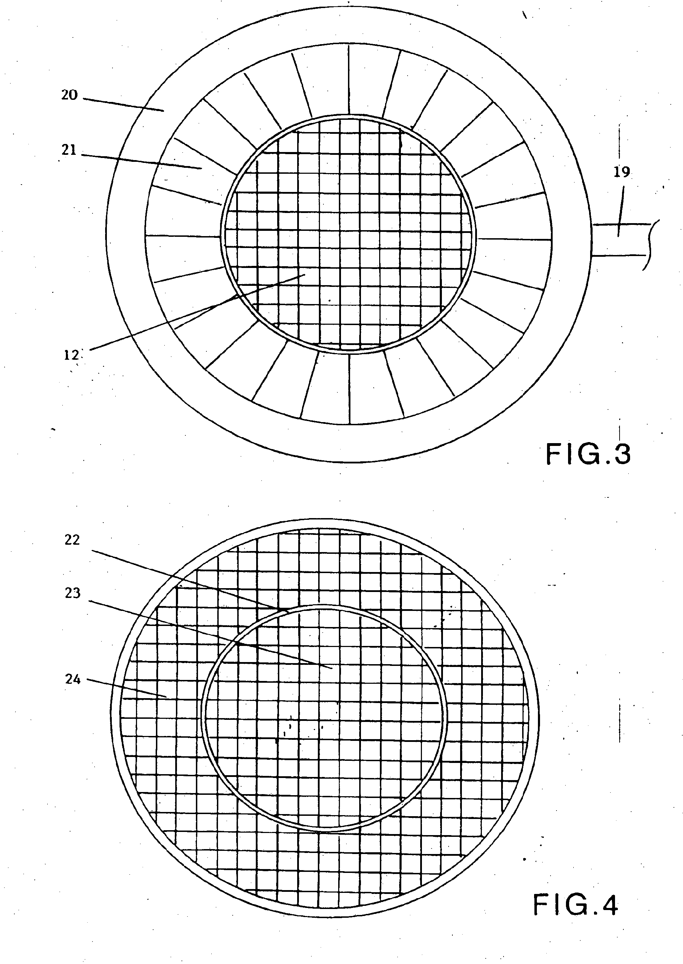

Fig.3 shows a view of the iris valve of a laminar flow reactor. In this picture is the iris holder provided with a handle to rotate the iris in positions to let through more or less fluid.

Fig.4 shows a reaktor with a grid 12 which is divided by a ring 22 in an inner part 23 where the cells are growing and an outer part 24 acting as bypass 17. Under the outer part 24 of the grid is situated a second grid (not shown) which can be rotated to covering the bypasses 17 partly or completly or to open the bypasses 17.

In this apparatus there is the possibillity to install an automatic controll for holding the laminar flow by measuring the pressure in the shell 9 behind the grid 12 by a measuring instrument 25 connected with the control box 7 connected with a not shown motor moving the handle 19 or another item to open and close the bypasses.

Also it is possible to install a device 26 for a simple take out the grid 12 with the grown cell mass and to bring in a new grid 12 for the growing cell mass. The apparatus with this device 26 is shown in Fig.4. Between the upper 9 and the under shell 8 is located this device 26. It consists of a holder 27 with rails 28 on its both sides to carry a slidable carrier 29 of the grid 12 for growing the cell masses and the surrounding bypasses 17. The carrier 29 has on its upper side and on its under side sealings 30 surrounding the grid 12 and the bypasses 17 to seal the carrier 29 with the shells 8.9 of the apparatus. The upper shell 9 is slidable against the under shell 8 in a direction of the flow of the nutrient fluid for a small distance to give the possibility to take out the carrier 29 and to fill in a new carrier 29. The magnitude of the diameter of the canals is controlled by the motor for adjusting 31. It can be hydraulic means which press the shells and the sealings together in the growing phase of the cell mass to tighten the apparatus and which lift the upper shell from the under shell at the end of the growing process to give a possibility to take out the carrier 29 out of the inner room between the two shells (it is the reactor room for the growing process) in an easy way. In the carrier 29 is a shaft 32 connecting the motor 31 with the ring of by-passes 17 to controll the passage of the bypasses 17. A handle 33 helps to slide the carrier 29.

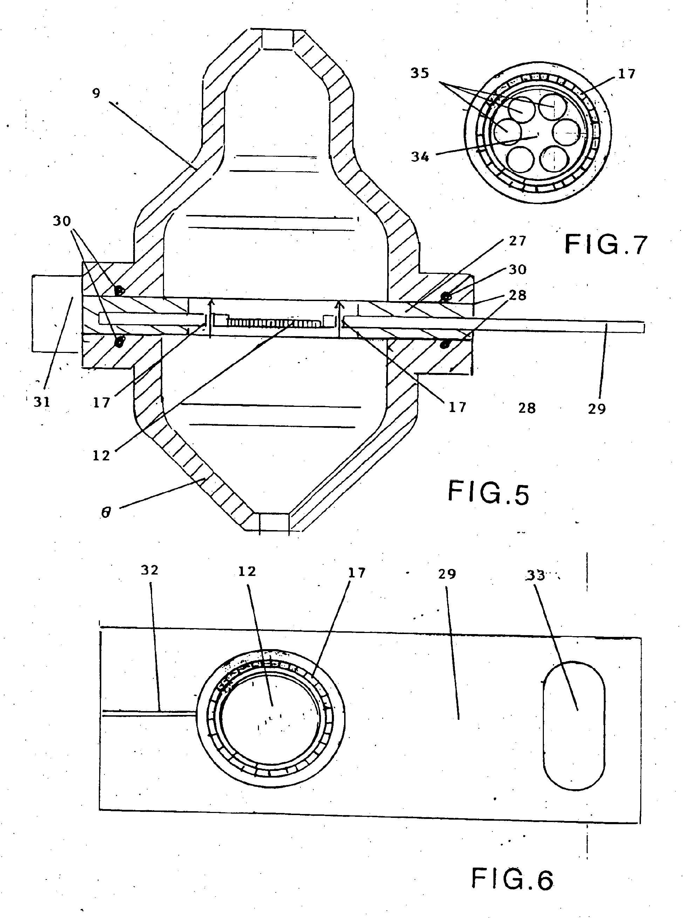

In Fig.7 shows a plate used as a shelter 34 with six holes 35. This shelter is to place on the grid 12 in the carrier if there is to observe the growth of cells in dependence with the time. In each hole is brought a little part of a cell mass. The carrier is brought in in the apparatus. After each day (or hour or another time) of the growing one hole is closed by a cover put over one hole, then the next hole and so on to interrupt the flow of the nutrient through this hole. In this way it is possible to observe the growth on the grid in the holes.

### List of the terms of reference

- 1: reactor
- 2: pump
- 3: pipe
- 4: pipe
- 5: storage tank
- 6: pipe
- 7: control box
- 8: shell
- 9: shell
- 10: interior of the shell
- 11: chamber
- 12: grid
- 13: hole (inlet)
- 14: hole (outlet)
- 15: flange
- 16: frame
- 17: canal for a bypass
- 18: ring
- 19: handle
- 20: iris holder
- 21: iris
- 22: ring
- 23: inner part of the grid
- 24: outer part of the grid
- 25: measure instrument
- 26: device
- 27: holder
- 28: rail
- 29: carrier
- 30: sealing
- 31: motor for adjusting
- 32: shaft
- 33: handle
- 34: plate used as a shelter
- 35: hole

## Claims

1. Method for the growth of cells to a three dimensional tissue, in which biomaterial is taken on a grid, the grid is put in the midst of a laminar flow of a nutrient and the grid is brought in the midst of a circle of bypasses, and in which the bypasses are closed or opened to control the flow to have a permanent laminar flow through the grid with its cells at the highest level without turbulences.

2. Apparatus for the culture and growth of cells to a three dimensional tissue, consisting of a laminar flow reactor, in which the growing of the cells takes place on a grid, a pump, a storage tank for a nutrient fluid and pipes connecting these parts to create a circulation of the nutrient fluid through these parts and with a plurality of bypasses controller by a handle or a motor to close the bypasses partly or completely to steer the run through of the nutrient fluid through the cell-mass.

3. Apparatus according claim 2, with a reactor whose housing consists of three parts, two shells each with a hole used for an entrance or an outlet of the nutrient fluid and between these shells a chamber carrying a grid to support the biomaterial.

4. Apparatus according claim 2 and 3, with a grid carrying support what is surrounded by a plurality of bypasses.

5. Apparatus according claim 4, with a support in form of a ring which has canals used as bypasses.

6. Apparatus according claim 5, with a grid in the form of a circle surrounded by the channels of the bypasses.

7. Apparatus according claim 2, with a slidable ring with the same scale of holes of the bypasses can be installed over the outlets or inlets of the bypasses to cover and close the bypasses partly or completely.

8. Apparatus according claim 2 and 3, with a control unit in form of a slidable ring over the plurality of bypasses to steering the flow through the bypasses by covering the bypasses partly or completely.

9. Apparatus according claim 2 to 5, with bypasses in form of an iris-diaphragm and an iris-clamp to control the flow through these bypasses.

10. Apparatus according claim 2 to 5, with shells having each a flange to fasten one shell to the other shell and including the grid bearing ring.

11. Apparatus according claim 2 to 5, with shells including a big volume of fluid to create an equal flow of the fluid through the chamber between the two shells.

12. Apparatus according claim 2 and 3, with a grid carrying a frame surrounding the center part of the grid for the growing of the cells and a surrounding part of the grid acting as a bypass.

13. Apparatus according claim 2 and 3, with a device for a simple take out the grid with the grown cell mass and to bring in a new grid for the growing cell mass located between the upper and the under shell consisting of the first hand of a holder to carry a slidable carrier with the grid for growing the cell masses and the surrounding bypasses and consisting on the second hand of an device for moving the upper and the under shell away from the holder (to open the apparatus for taking out the grid with the grown cell mass) and to press the two shells against the holder (to close the apparatus for its working phase).

## Patentansprüche

1. Verfahren für das Wachsen von Zellen zu einem dreidimensionalen Gewebe, bei dem Biomaterial auf ein Gitternetz gebracht wird, das Gitternetz in die Mitte einer laminaren Strömung eines Nährstoffs gesetzt wird und das Gitternetz in die Mitte eines Kreises von Nebenschlüssen überführt wird und bei dem die Nebenschlüsse geschlossen oder geöffnet werden, um die Strömung zu kontrollieren, um eine permanente laminare Strömung durch das Gitternetz mit seinen Zellen auf dem höchsten Niveau ohne Turbulenzen zu erhalten.

2. Apparat zur Kultur und zum Wachsen von Zellen zu einem dreidimensionalen Gewebe, der aus einem laminaren Strömungsreaktor besteht, bei dem das Wachsen der Zellen auf einem Gitternetz stattfindet, einer Pumpe, einem Aufbewahrungstank für eine Nährflüssigkeit und Schläuchen, welche diese Teile miteinander verbinden, um eine Zirkulation der Nährflüssigkeit durch diese Teile herzustellen und mit einer Vielzahl von Nebenschlüssen, die von einem Hebel oder einem Motor kontrolliert werden, um die Nebenschlüsse teilweise oder vollständig zu schließen, um den Durchlauf der Nährflüssigkeit durch die Zellmasse zu regulieren.

3. Apparat nach Anspruch 2 mit einem Reaktor, dessen Gehäuse aus drei Teilen besteht, jeweils zwei Mänteln mit einem Loch, das für einen Zufluss oder einen Abfluss der Nährflüssigkeit verwendet wird und zwischen den Mänteln einer Kammer, die ein Gitternetz zum Stützen des Biomaterials trägt.

4. Apparat nach Anspruch 2 und 3 mit einer das Gitternetz tragenden Auflage, die von einer Vielzahl von Nebenschlüssen umgeben ist.

5. Apparat nach Anspruch 4 mit einer Auflage in Form eines Rings, der Kanäle aufweist, die als Nebenschlüsse verwendet werden.

6. Apparat nach Anspruch 5 mit einem Gitternetz in Form eines Kreises, der von den Kanälen der Nebenschlüsse umgeben ist.

7. Apparat nach Anspruch 2, wobei ein verschiebbarer Ring mit derselben Skalierung von Löchern der Nebenschlüsse über den Abflüssen oder Zuflüssen der Nebenschlüsse installiert werden kann, um die Nebenschlüsse teilweise oder vollständig zu bedecken und zu verschließen.

8. Apparat nach Anspruch 2 und 3 mit einer Kontrolleinheit in Form eines über die Vielzahl von Nebenschlüssen verschiebbaren Rings, um die Strömung durch die Nebenschlüsse durch das teilweise oder vollständige Bedecken der Nebenschlüsse zu regulieren.

9. Apparat nach Anspruch 2 bis 5 mit Nebenschlüssen in Form eines Irisdiaphragmas und einer Irisklemme, um die Strömung durch die Nebenschlüsse zu kontrollieren.

10. Apparat nach Anspruch 2 bis 5 mit Mänteln, die jeweils einen Flansch aufweisen, um einen Mantel an dem anderen Mantel zu befestigen und die den das Gitternetz tragenden Ring umfassen.

11. Apparat nach Anspruch 2 bis 5 mit Mänteln, die ein großes Volumen an Flüssigkeit umfassen, um eine gleichmäßige Strömung der Flüssigkeit durch die Kammer zwischen den beiden Mänteln herzustellen.

12. Apparat nach Anspruch 2 und 3 mit einem Gitternetz, das einen Rahmen trägt, der den zentralen Anteil des Gitternetzes für das Wachsen der Zellen umgibt und einen umliegenden Anteil des Gitternetzes, der als ein Nebenschluss wirkt.

13. Apparat nach Anspruch 2 und 3 mit einer Vorrichtung zur einfachen Entnahme des Gitternetzes mit der gewachsenen Zellmasse und zum Einbringen eines neuen Gitternetzes für die wachsende Zellmasse, die sich zwischen dem oberen und dem unteren Mantel befindet, die einerseits aus einem Halter besteht zum Tragen eines verschiebbaren Trägers mit dem Gitternetz für das Wachsen der Zellmassen und den umliegenden Nebenschlüssen und andererseits aus einer Vorrichtung besteht zum Bewegen des oberen und des unteren Mantels von dem Halter hinweg (zum Öffnen des Apparats, um das Gitternetz mit der gewachsenen Zellmasse zu entnehmen) und um die zwei Mäntel gegen den Halter zu drücken (zum Schließen des Apparats für dessen Arbeitsphase).

## Revendications

1. Procédé de croissance de cellules en un tissu en trois dimensions, dans lequel le biomatériau est pris sur une grille, la grille est placée dans la brume d'un flux laminaire d'un nutriment et la grille est amenée dans la brume d'un cercle de dérivations, et dans lequel les dérivations sont fermées ou ouvertes pour commander le flux en vue d'avoir un flux laminaire permanent à travers la grille avec ses cellules au plus haut niveau sans turbulence.

2. Appareil pour la culture et la croissance de cellules en un tissu en trois dimensions, constitué d'un réacteur à flux laminaire, dans lequel la croissance des cellules a lieu sur une grille, d'une pompe, d'une cuve de stockage pour fluide nutritif et de tubes raccordant ces parties pour créer une circulation du fluide nutritif à travers ces parties et avec une pluralité de dérivations commandées par une poignée ou un moteur pour fermer partiellement ou complètement les dérivations et orienter l'écoulement du fluide nutritif à travers la masse de cellules.

3. Appareil selon la revendication 2, avec un réacteur dont le boîtier est constitué de trois parties, deux coques ayant chacune un trou utilisé pour une entrée ou un orifice de sortie du fluide nutritif et entre ces coques une chambre portant une grille pour supporter le biomatériau.

4. Appareil selon les revendications 2 et 3, avec un support portant une grille qui est entouré d'une pluralité de dérivations.

5. Appareil selon la revendication 4, avec un support de forme annulaire qui a des canaux utilisés en tant que dérivations.

6. Appareil selon la revendication 5, avec une grille sous la forme d'un cercle entourée par les canaux des dérivations.

7. Appareil selon la revendication 2, dans lequel un anneau coulissant avec la même échelle que les trous des dérivations peut être installé par-dessus les orifices de sortie ou les orifices d'entrée des dérivations pour couvrir et fermer partiellement ou complètement les dérivations.

8. Appareil selon les revendications 2 et 3, avec une unité de commande sous la forme d'un anneau coulissant par-dessus la pluralité de dérivations pour orienter le flux à travers les dérivations par couverture partielle ou complète des dérivations.

9. Appareil selon les revendications 2 à 5, avec des dérivations en forme d'un diaphragme à iris et une pince à iris pour commander le flux dans ces dérivations.

10. Appareil selon les revendications 2 à 5, les coques ayant chacune un rebord pour fixer une coque à l'autre coque et comprenant l'anneau portant la grille.

11. Appareil selon les revendications 2 à 5, les coques comprenant un gros volume de fluide pour créer un flux égal du fluide à travers la chambre entre les deux coques.

12. Appareil selon les revendications 2 et 3, avec une grille portant un châssis entourant la partie centrale de la grille pour la croissance des cellules et une partie environnante de la grille servant de dérivation.

13. Appareil selon les revendications 2 et 3, avec un dispositif permettant de retirer simplement la grille avec la masse de cellules développées et de placer une nouvelle grille pour la croissance de la masse cellulaire située entre les coques supérieure et inférieure constitué de la première poignée d'une monture pour porter un support coulissant avec la grille pour développer les masses cellulaires et les dérivations environnantes et constitué de la seconde poignée d'un dispositif destiné à éloigner les coques supérieure et inférieure de la monture (pour ouvrir l'appareil et retirer la grille avec la masse de cellules développées) et presser les deux coques contre la monture (pour fermer l'appareil pour sa phase de travail).
